# EUROPÄISCHE PATENTANMELDUNG

(11) **EP 0 623 326 A1**
(43) Veröffentlichungstag der Anmeldung: **09.11.1994**
(21) Anmeldenummer: 93810337.1
(22) Anmeldetag: 07.05.1993
(51) Int. Cl.: A61F 5/41, A61F 6/04

(54) **Kondom**

(71) Anmelder: Cimber, Hugo, Dr. med., CH-3072 Ostermundigen (CH)
(72) Erfinder: Cimber, Hugo, Dr. med., CH-3072 Ostermundigen (CH)
(74) Vertreter: Fischer, Franz Joseph

(57) **Zusammenfassung**

Das Kondom umfasst neben einer länglich ausgebildeten Hülle (1) aus gummielastischem filmartigem Material und an seiner Oeffnung (3) einen Spannungsring (4), welcher der Ueberwindung von Potenzschwächen dient. Zur Applikation ist ein Hilfsmittel (5) vorgesehen, das im wesentlichen die Form eines Kegels besitzt. Dazu gehört ein auf die Basis des kegelförmigen Teils passender starrer Ring (6) mit einer Nut (8) auf welchen der Spannungsring (4) vor der Anwendung des Kondoms vorgespannt werden kann.

## Beschreibung

Die vorliegende Erfindung betrifft ein Kondom, welches gleichzeitig ein Verhütungsmittel, eine Barriere gegen die Übertragung von Krankheiten und ein Hilfsmittel gegen Potenzschwäche darstellt.

Nach dem Siegeszug der empfängnisverhütenden Pillen in den 60er- und 70er Jahren wurde das Kondom als Verhütungsmittel immer mehr in den Hintergrund gedrängt. Erst in den 80er Jahren mit dem Aufkommen der AIDS-Problematik erhielten die Präservative eine erneute Bedeutung. Es steht jedoch nicht mehr die Empfängnisverhütung, sondern der Schutz vor einer AIDS-Infektion im Vordergrund. Präservative werden deshalb von ärztlichen wie auch von staatlichen Institutionen seit längerer Zeit den Ansteckungsgefährdeten dringend empfohlen, wobei hierzu Gebrauch von sämtlichen zur Verfügung stehenden Werbemedien gemacht wird.

Mit dem Verschwinden von vielen Tabus auf dem Gebiet der Sexualität und auch mit der steigenden Lebenserwartung sehen sich immer mehr Spezialärzte mit den Problemen der sexuellen Dysfunktion wie Erektionsstörungen von Männern konfrontiert. Dabei ist nicht immer leicht auszumachen ob es sich bei den Störungen um solche von psychischer oder organischer Ursache handelt. Die Medizin kennt heute zur Behebung der Erktionsschwäche verschiedene Hilfsmittel, wie Injektion von vasoaktiven Substanzen, Implantation von Prothesen und Unterstützung des Einströmens von arteriellem Blut in die Schwellkörper des Penis durch Unterdruck mittels einer Handpumpe. Es gibt jedoch viele Patienten, die einen chirurgischen Eingriff oder auch die Injektion eines Wirkstoffes ablehnen und sich auf einfache Hilsmittel beschränken möchten. Es ist sei langem bekannt, dass bei Erektionsschwäche eine Erektion verlängert werden kann, indem das Zurückströmen von Blut aus dem Glied während einer gewissen Zeit durch eine vorübergehende abschnürende Wirkung eines Spannungsringes aus Gummi verhindert wird. Ein derartiger Spannungsring ist beispielsweise in der US-A-4 856 498 beschrieben, wo seine Verwendung in Kombination mit einem Vakuumgerät vorgeschlagen wird.

Es ist demzufolge Aufgabe der vorliegenden Erfindung ein Mittel zur Verfügung zustellen, das auf mechanische Weise gleichzeitig ein Hilfsmittel gegen Potenzschwäche, ein Schutzmittel gegen Übertragung des HIV-Virus und auch ein Verhütungsmittel darstellt.

Die Aufgabe wurde erfindungsgemäss durch ein Kondom gelöst, das eine länglich ausgebildete Hülle aus gummielastischem filmartigem Material, mit einem offenen und geschlossenen Ende aufweist, wobei am offenen Ende eine durch den Hüllenrand gebildete Öffnung vorhanden ist und der Hüllenrand um die Öffnung als elastischer Ring ausgebildet ist, welches Kondom dadurch gekennzeichnet ist, dass der elastische Ring ein Spannungsring ist, dessen innerer Durchmesser der Grösse des Umfangs der Basis des Penis derart angepasst ist, dass der Blutabfluss aus den arteriellen Gefässen im erigierten Glied gehemmt wird.

Der Spannungsring weist vorzugsweise eine torusförmige Gestalt auf und besteht aus einem gummielastischen Material mit eine Shorehärte A von ca. 40. Er ist vorteilhafterweise mit zwei oder mehr Griffen zu seiner Handhabung versehen. Diese Griffe können die Form von Ringen, Lappen und dergleichen aufweisen.

Da wegen dem Spannungsring die Handhabung eines erfindungsgemässen Präservativs von derjenigen eines gewöhnlichen Kondoms verschieden ist, sollte einerseits seine Gummihaut eine genügende Weite und Stabilität aufweisen und mit einem Gleitmittel behandelt sein, damit ein Überziehen im nicht aufgerollten Zustand problemlos möglich ist. Andernseits muss der Spannungsring für seine Anwendung auf geeignete Weise vorgespannt werden. Hierzu ist ein Applikationshilfsmittel vorgesehen, das einen starren Ring umfasst, der an seiner äusseren Seite in äquatorialer Richtung eine Einkerbung oder Nut besitzt. Der Spannungsring des Kondoms wird vor der Verwendung über den starren Ring gespannt, wobei er in die Einkerbung oder Nut zu liegen kommt, damit eine zur Applikation genügend grosse Öffnung mit einem Durchmesser von ca. 50 mm gebildet wird. Vorteilhafterweise ist die Einkerbung derart ausgebildet, dass die Kerbe des Spannungsring in proximaler Richtung durch einen höheren Rand begrenzt ist als der in distaler Richtung liegende Rand. Sobald sich das Kondom an der vorgesehenen Stelle befindet, kann der starre Ring in proximaler Richtung herausgedrückt, und in distaler Richtung über das Kondom entfernt werden.

Das Kondom kann mit gespanntem Spannungsring, d.h. mit einem eingesetzten starren Ring, vom Hersteller geliefert werden, wenn die Verbrauchsfrist des Produktes so angesetzt wird, dass die Spannkraft des Spannungsrings bei Entfernung des starren Rings bezüglich des ursprünglichen Zustands nicht merklich nachlässt bzw. die Anforderungen erfüllt. Alternativ kann das Produkt vom Hersteller ebenfalls mit einem nicht gespannten Spannungsring vertrieben werden. Für die Applikation ist hierzu jedoch erforderlich, dass ein Hilfsmittel zu seiner Spannung beigefügt wird. Hierzu wurde neben dem starren Ring ein kegel- oder kegelstumpfförmiges Teil vorgesehen, das im Zusammenspiel mit dem erwähnten starren Ring dazu dient, das Kondom in eine anwendungsgerechte Form zu bringen. Zur Anwendung wird der starre Ring, der eine den Ring aussen umfassende Nut aufweist, auf das kegel- oder kegelstumpfförmige Teil des Applikationshilfsmittels aufgesetzt. Dieses ist derart ausgestaltet, dass der starre Ring, wenn er über das kegelförmige Teil gezogen wird, in der Nähe seiner Basis anstösst oder leicht einrastet, damit der starre Ring nicht über die Basis weggleiten kann. Das Kondom kann auf das konische Teil aufgeschoben werden, wobei der Spannungsring so weit gedehnt wird, dass er in die Nut auf dem starren Ring zu liegen kommt. Das Kondom kann nun mit gespanntem Spannungsring vom konischen Teil des Applikationshilfsmittels entfernt werden, wobei der starre Ring im Spannungsring sitzen bleibt und das Kondom anwendungsbereit ist.

Die Materialien für Kondome sind den Fachleuten bekannt und umfassen synthetische und natürliche Gummiarten. Die Anbringung des Spannungsringes an die Hülle des Kondoms kann nach den üblichen Techniken der Gummiverarbeitung, beispielsweise durch Vulkanisation, erfolgen. Die Applikationshilfsmittel werden in der Regel aus gängigen Kunststoffmaterialien hergestellt, welche die mechanischen Anforderungen erfüllen, wie z.B. Polypropylen, Polymethylen, Polycarbonat.

Die Erfindung wird durch die nachstehenden Zeichnungen, die nur eine einzige Ausführungform darstellen, näher erläutert. Es zeigt.
Fig. 1 einen Längsschnitt durch ein Kondom gemäss der der Erfindung,
Fig. 2 einen Schnitt durch ein Applikationshilfsmittel für das Kondom,
Fig. 3 eine Vorderansicht des Applikationshilfsmittels gemäss Fig.2,
Fig. 4 einen Teilschnitt durch ein Applikationshilfsmittel mit aufgesetzem Kondom,
Fig. 5 ein Spannungsring, wie er am Kondom zur Anwendung gelangt, und
Fig. 6 eine perspektivische Ansicht eines zusammenlegbaren Applikationshilfsmittels, das in zwei Segmente aufgeteilt ist.

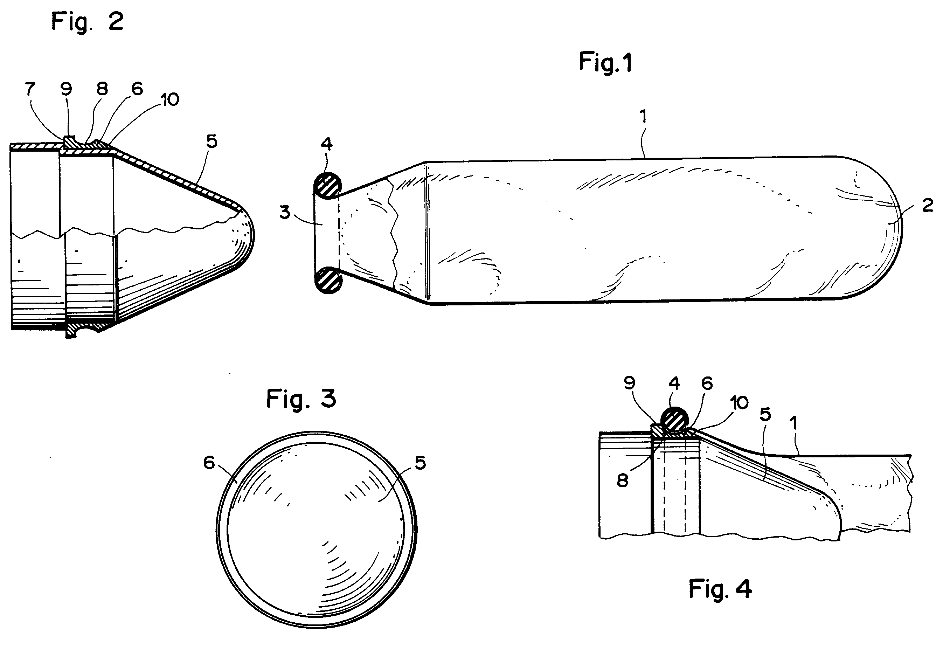

Fig. 1 zeigt einen Schnitt durch ein Kondom gemäss der Erfindung, welches eine Hülle 1 aufweist, die ein geschlossenes Ende 2 und ein offenes Ende 3 aufweist. Am offenen Ende ist ein Spannungsring 4 angeordnet. Der innere Durchmesser des Spannungsrings beträgt vorzugsweise ca. 20 mm. Der Materialdurchmesser des Ringes beträgt vorteilhaft ca. 6,5 mm.

Fig. 2 zeigt einen Schnitt durch ein Applikationshilfsmittel bestehend aus einem im wesentlichen kegelförmigen Teil 5 und einem darauf aufgesetzten starren Ring 6. Dieser besteht vorteilhafterweise aus einem im wesentlichen starren Kunststoffmaterial, das eine genügende Stärke aufweist, um den Spannungsring 4 in gespanntem Zustand ausgedehnt auf einem inneren Durchmesser von ca. 50 mm zu tragen. Das Applikationshilfsmittel weist in der Nähe der Basis einen Anschlag 7 auf, damit der starre Ring 6 nicht über die Basis hinaus weggeschoben werden kann. Der starre Ring 6 weist eine Nut 8 auf, welche zum Aufnehmen des Spannungsrings 4 in gespanntem Zustand dient. Diese Nut besitzt in Richtung der Basis des Applikationshilfsmittels, bzw. in proximaler Richtung bezogen auf den Anwender, einen höheren Rand 9, damit der Spannungsring 4 nicht unbeabsichtigt über diesen Rand 9 hinausgeschoben werden kann. In Richtung des verjüngten Teil des Applikationshilfsmittel ist ein weniger hoher Rand 10 vorhanden, über welchen der Spannungsring 4 leicht in die Nut 8 hineingeschoben werden kann.

Fig. 3 zeigt eine Vorderansicht des Applikationshilfsmittels 5, wobei die Lage des starren Rings 6 sichtbar ist.

Fig. 4 ist ein Teilschnitt durch ein Applikationshilfsmittel 5 mit aufgesetztem Kondom mit Spannungsring 4 und Hülle 1. Hier ist ersichtlich, dass sich der Spannungsring 4 auf dem starren Ring 6 in der Nut 8 befindet, wobei der höhere Rand 9 verhindert, dass der Spannungsring unbeabsichtigt in die Innenseite des Kondoms geschoben werden kann.

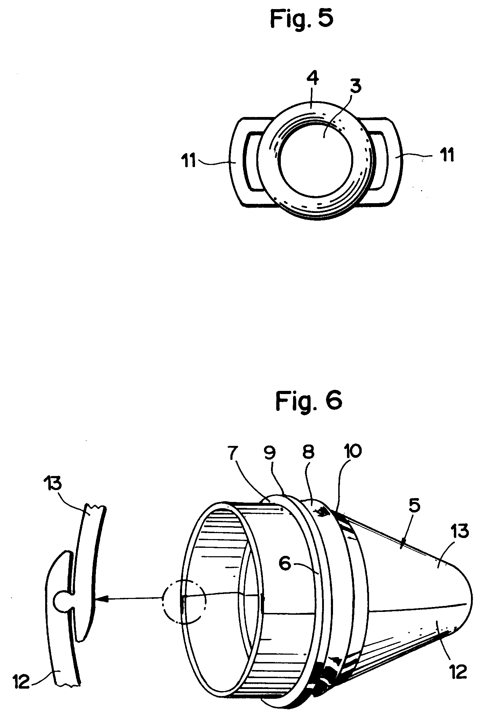

Fig. 5 zeigt die Ansicht eines Spannungsrings 4, welcher an seiner Aussenseite gegenüberliegende Handgriffe 11 aufweist. Diese erleichtern das Aufsetzen des Kondoms auf das Applikationshilfsmittel und erlauben ebenfalls die Handhabung nach der Anwendung. Der Spannungsring 4 ist vorzugsweise aus Kautschuk oder synthetischem Gummi und soll eine Shore-Härte A von 40 besitzen.

Schliesslich zeigt Fig. 6 eine perspektivische Ansicht eines Teil des Applikationshilfsmittels, das im wesentlichen die Form eines Kegels besitzt. Dieses Hilfsmittel ist in 2 Segmente 12 und 13 aufgeteilt, und kann zur Platzeinsparung zerlegt oder zusammengeklappt werden. Die beiden Segmente sind für die Anwendung durch eine einfache Kupplungsvorrichtung verbunden und können nach seiner Anwendung wiederum in die beiden Segmente zerlegt werden. Ebenfalls sichtbar ist die Kante 7, an welcher der starre Ring 6 anschlägt, damit er nicht über die Basis des konischen Teils abrutschen kann.

## Patentansprüche

1. Kondom umfassend eine länglich ausgebildete Hülle (1) aus gummielastischem filmartigem Material, mit einem offenen und geschlossenen Ende (3, 2), wobei am offenen Ende (3) eine durch den Hüllenrand gebildete Öffnung vorhanden ist und der Hüllenrand um die Öffnung als elastischer Ring ausgebildet ist, dadurch gekennzeichnet, dass der elastische Ring ein Spannungsring (4) ist, dessen innerer Durchmesser der Grösse des Umfangs der Basis des Penis derart angepasst ist, dass der Blutabfluss von den arteriellen Gefässen im erigierten Glied gehemmt wird.

2. Kondom nach Anspruch 1 dadurch gekennzeichnet, dass der Spannungsring (4) toroidförmig ist.

3. Kondom nach Anspruch 1 oder 2, dadurch gekennzeichnet, dass der Spannungsring an seiner äusseren Seite mindestens zwei Griffe (11) aufweist.

4. Kondom nach Anspruch 3. dadurch gekennzeichnet, dass die Griffe (11) ring- oder lappenförmig sind.

5. Kondom nach einem der Ansprüche 1 bis 4, dadurch gekennzeichnet, dass Hülle (1) eine genügende Weite aufweist, damit ein beschädigungsfreies Überziehen ohne Abrollen möglich ist.

6. Applikationshilfsmittel für einen Kondom nach einem der Ansprüche 1 bis 5, dadurch gekennzeichnet, dass es einen starren Ring (6) besteht, der an seiner äusseren Seite in äquatorialer Richtung eine Einkerbung oder Nut (8) besitzt, derart, dass der Spannungsring (4) des zu applizierenden Kondoms, wenn er über den starren Ring (6) gespannt wird, in die Einkerbung oder Nut (8) zu liegen kommt, damit eine zur Applikationn genügend grosse Öffnung gebildet wird.

7. Applikationshilfsmittel nach Anspruch 6, dadurch gekennzeichnet, dass die Einkerbung oder Nut (8) derart ausgebildet ist, dass diese in proximaler Richtung durch einen höheren Rand (9) begrenzt ist als in distaler Richtung, derart, dass der starre Ring (6), sobald sich das Kondom an der vorgesehenen Stelle befindet, herausgedrückt, und in distaler Richtung über das Kondom entfernt werden kann.

8. Applikationshilfsmittel nach Anspruch 6 oder 7, dadurch gekennzeichnet, dass es weiter ein kegel- oder kegelstumpfförmiges Teil (5) umfasst, das in solcher Weise in den starren Ring (6) hinein passt, dass der starre Ring das kegel- oder kegelstumpfförmige Teil (5) an dessen Basis umfasst, derart, dass das Kondom mit dem Spannungsring über das kegel- oder kegelstumpfförmige Teil gezogen werden kann und der Spannungsring auf den starren Ring zu sitzen kommt, und das kegelförmige Teil (5) ohne den starren Ring (6) wieder entfernt werden kann.

9. Applikationshilfsmittel nach Anspruch 8 dadurch gekennzeichnet, dass das kegel- oder kegelstumpfförmige Teil (5) in mindestens zwei Segmente (12, 13) zerlegbar ist.
